# EUROPÄISCHE PATENTSCHRIFT

(11) **EP 1 567 081 B1**
(45) Veröffentlichungstag und Bekanntmachung des Hinweises auf die Patenterteilung: **23.04.2008**
(21) Anmeldenummer: 03776897.5
(22) Anmeldetag: 11.11.2003
(51) Int. Cl.: A61B 18/14, A61B 17/22

(54) **METALLELEKTRODE**
METAL ELECTRODE
ELECTRODE METALLIQUE

(30) Priorität: 11.11.2002 DE 10252325; 06.12.2002 DE 10257146
(43) Veröffentlichungstag der Anmeldung: 31.08.2005
(73) Patentinhaber: Admedes Schuessler GmbH, 75179 Pforzheim (DE); Berchtold Holding GmbH, 78532 Tuttlingen (DE)
(72) Erfinder: FLAXMEIER, Erik, 76307 Karlsfeld (DE); STEINER, Ralf, 75181 Pforzheim (DE); MÜLLER, Wolfgang, D-72131 Ofterdingen (DE)
(74) Vertreter: Müller-Boré & Partner Patentanwälte
(86) Internationale Anmeldenummer: PCT/EP2003/012600
(87) Internationale Veröffentlichungsnummer: WO 2004/043279

(56) Entgegenhaltungen:
- WO-A-01/58382
- US-A- 5 681 280
- US-A- 5 725 525
- US-A- 5 730 128
- US-A1- 2002 103 501
- US-B1- 6 319 251
- US-B1- 6 391 044

## Beschreibung

Die Erfindung betrifft eine Spreizstruktur zum Einführen in ein Hohlorgan, mit Spreizstäben, die sich ausgehend von einem ersten Verbindungsabschnitt im Wesentlichen in einer Längsrichtung der Spreizstruktur bis zu einem zweiten Verbindungsabschnitt erstrecken, über den Umfang der Spreizstruktur verteilt angeordnet sind und durch radiales Aufspreizen an eine Wand des Hohlorgans anlegbar sind. Ferner betrifft die Erfindung eine Verwendung der Spreizstruktur, eine Spreizvorrichtung und ein Verfahren zum Anordnen einer Spreizstruktur.

Spreizstrukturen der oben genannten Art werden als Implantate für einen begrenzten Zeitraum oder auf Dauer in ein Hohlorgan, wie beispielsweise Herz, Blutgefäße, Gallenwege, ableitende Harnwege, Magen-Darm-Trakt, Uterus und Hirnventrikel, eingesetzt, um beispielsweise Thromben oder Gallengangssteine zurückzuhalten. So sind verschiedene Arten von Thrombosefilter bekannt, die als Venenimplantat perkutan über die Vena femoralis oder die Vena jugularis in die Vena cava inferior oder die Vena cava superior eingeführt werden. Dort soll das Venenimplantat Thromben auf ihrem Weg zum Herzen zurückhalten und dadurch einer Lungenembolie vorbeugen. Solche Implantate sind in der Regel mit konisch verlaufenden Streben oder Stäben gestaltet, die einen trichterförmigen Filterkorb bilden.

Ferner sind selbstexpandierende Endoprothesen zum Offenhalten von gangartigen Strukturen bekannt, die als sogenannte Stents auch in Blutgefäßen zum Einsatz kommen. Stents sind in der Regel mit einem schlauchförmigen, mehr oder weniger feinmaschigen Drahtgewebe oder Drahtgeflecht gestaltet, das unter einer elastischen Rückstellkraft seiner Drähte radial expandierbar ist.

US 5,681,280 offenbart einen lenkfähigen Katheter mit einem elastisch ablenkbaren Körperelement. Das ablenkbare Körperelement weist ein expandierfähiges Elektrodenfeld an einem distalen Ende auf. Das Elektrodenfeld umfasst eine Vielzahl von peripheral verlängerten Segmenten, die flexibel und elastisch sind. Insbesondere weisen die Segmente eine im Wesentlichen gerade bzw. glatte Form auf, wenn keine Biegungskraft auf sie ausgeübt wird.

Der Erfindung liegt die Aufgabe zugrunde, eine verbesserte Spreizstruktur zur Verfügung zu stellen, die sehr präzise ist, leicht komprimiert und expandiert werden kann.

Diese Aufgabe wird gemäß einer Spreizstruktur mit den Merkmalen nach Anspruch 1 gelöst. Vorteilhafte Weiterbildungen sind in den abhängigen Ansprüchen definiert.

Erfindungsgemäß ist aus Spreizstäben ein Körper bzw. eine Spreizstruktur gestaltet, der in seiner Ruhelage oder in einem vorgespannten Zustand im Wesentlichen zylindrisch gestaltet ist. Dafür kann die erfindungsgemäße Spreizstruktur in ihrer Ruhestellung länglich geformt sein und aus dieser Ruhestellung heraus gespreizt werden, oder sie kann in ihrer Ruhestellung eine ballonartige Form aufweisen und aus dieser Ruhestellung heraus in eine längliche Form komprimiert werden.

Bei dem erfindungsgemäßen Körper haben insbesondere die Spreizstäbe in Längsrichtung zumindest einen Bereich, der im Vergleich zu benachbarten Bereichen eine verringerte Biegesteifigkeit aufweist

Die erfindungsgemäße Spreizstruktur kann sehr präzise und zugleich leicht komprimiert bzw. expandiert werden. Durch die erfindungsgemäß vorgesehenen Bereiche verringerter Biegesteifigkeit ist an den einzelnen Stäben eine Sollknickstelle gebildet, an der sich der Stab beim Zusammenziehen bzw. Komprimieren und Spreizen bzw. Expandieren definiert verformt

Die erfindungsgemäße Spreizstruktur kann zum Weiten und Offenhalten eines Gefäßes verwendet werden oder auch als Fangkörbchen beispielsweise für Blasensteine, Harnleitersteine, Nierensteine sowie für Gallengangsteine dienen.

Darüber hinaus können durch das definierte Expandieren und Zusammenziehen der erfindungsgemäßen Spreizstruktur Fremdkörper im Ösophagus entfernt werden.

Die Spreizstruktur gemäß der Erfindung findet daher vorteilhaft sowohl in der Urologie als auch in der Gastroenterologie Anwendung.

Schließlich kann die erfindungsgemäße Spreizstruktur auch im Bereich des peripheren und des coronaren Blutsystems verwendet werden. So kann die Spreizstruktur als Distal Embolic Protection oder als temporärer Vena Cava Filter dienen. Ferner kann mit der Erfindung ein Septal Okkluder System oder ein Aneurysma Okklusions System besonders vorteilhaft realisiert werden.

Die erfindungsgemäße Spreizstruktur kann darüber hinaus als Metallelektrode verwendet werden, um an einer Wand des Hohlorgans Wärme einzubringen und dort beispielsweise eine Elektrokoagulation durchzuführen.

Bei einer vorteilhaften Weiterbildung der Erfindung ist der mindestens eine Bereich mit verringerter Biegesteifigkeit im Vergleich zu den benachbarten Bereichen mit einem verringerten Querschnitt ausgebildet Dabei wird innerhalb der einzelnen Bereiche vorteilhaft ein und dasselbe Material verwendet, so dass die Spreizstruktur insgesamt aus einem einzelnen Material hergestellt sein kann. Als Material eignet sich besonders ein nicht korrodierendes Metall, insbesondere eine Edelstahllegierung. Besonders sinnvoll sind ferner Tantal, Niob, Kobaltlegierungen und andere Werkstoffe, z.B. Polymere, selbstabbaubare Werkstoffe (z.B. Milchsäure-Werkstoffe bzw. -Derivate). Dabei kann eine fremdexpandierende Spreizstruktur (insbesondere aus einer Nickel-TitanLegierung, vor allem aus Nitinol) oder eine selbstexpandierende Spreizstruktur (insbesondere aus einer Form-Gedächnis-Legierung) gebildet sein. Das Material kann elektrochemisch poliert oder beschichtet sein. Der Bereich mit dem verringerten Querschnitt erwärmt sich bei Verwendung der Spreizstruktur als Metallelektrode besonders stark und führt daher zu einem gezielten Wärmeeintrag an der Wand des Hohlorgans.

Der Bereich mit verringerter Biegesteifigkeit ist mit einem nicht linearen Stababschnitt gestaltet, der bereits Biegungen oder Knicke aufweist und daher beim Spreizen der Spreizstäbe besonders stark verformt wird. Der nicht lineare Stababschnitt führt ferner zu einer besonders intensiven Verankerung der erfindungsgemäßen Spreizstruktur an der Wand des behandelten Hohlorgans.

Für den erfindungsgemäßen Bereich mit verringerter Biegesteifigkeit eignet sich ferner insbesondere eine mäanderförmige Struktur. Eine Mäanderstruktur ist durch abwechselnde gebogene und geradlinige Abschnitte gekennzeichnet, die sich in einer Fläche erstrecken. Die Fläche kann eben sein oder wie im vorliegenden Fall bevorzugt die Mantelfläche eines Kreiszylinders sein. Dies bedeutet, dass für die erfindungsgemäße Spreizstruktur ein Rohr als Ausgangselement dient, aus dem die Spreizstäbe beispielsweise durch Laserschneiden herausgeschnitten werden. Die Mäanderstruktur ermöglicht eine besonders leichte Verformung senkrecht zur Fläche der Mäander. Es ist daher ferner besonders vorteilhaft, wenn die Fläche der Mäander eine Tangentenebene an den Umfang der Spreizstruktur bildet.

Für den erfindungsgemäßen Bereich mit verringerter Biegesteifigkeit sind ferner die Wellenform oder die Zickzackform besonders geeignet. Die Wellenform ist durch aufeinanderfolgende im Wesentlichen jeweils abwechselnd gekrümmte Abschnitte gekennzeichnet, während die Zickzackform durch aufeinanderfolgende im Wesentlichen gerade Abschnitte gekennzeichnet ist, die an punktartigen Knickstellen aneinanderstoßen.

Um eine gezielte Verformung der Spreizstäbe in definierten Richtungen zu erzielen ist es ferner vorteilhaft, wenn der Bereich der verringerten Biegesteifigkeit im Querschnitt quadratisch gestaltet.

Die Verbindungsabschnitte sind besonders vorteilhaft als zentrale Knotenpunkte im Bereich der Längsachse der Spreizstruktur ausgebildet. Die Verbindungsabschnitte bilden so an den Enden der Spreizstruktur, insbesondere an dem distalen Ende, je eine "Spitze", die ein Einführen, Fortbewegen und Herausführen der Spreizstruktur im Hohlorgan erleichtert.

Ferner ist in mindestens einem der Verbindungsabschnitte eine Öffnung ausgebildet, durch die hindurch ein zentraler Stab bis zu dem zweiten Verbindungsabschnitt hindurchgeführt werden kann. Mit dem zentralen Stab kann die erfindungsgemäße Spreizstruktur expandiert und komprimiert werden, indem der Abstand entlang der Längsachse der Spreizstruktur zwischen den Verbindungsabschnitten verringert bzw. vergrößert wird.

Die Verbindungsabschnitte sind besonders vorteilhaft im Wesentlichen zylindrisch gestaltet, so dass die Spreizstäbe regelmäßig verteilt an ihrem Umfang angeordnet sein können und somit insgesamt eine über den Umfang der Spreizstruktur gleichmäßig stabile Struktur geschaffen ist

Im Ausgangszustand der erfindungsgemäßen Spreizstruktur weisen die Spreizstäbe vorteilhaft jeweils ausgehend von einem Verbindungsabschnitt einen radial nach außen gebogenen ersten Abschnitt und einen nachfolgenden im Wesentlichen geraden zweiten Abschnitt auf. An dem vorgebogenen ersten Abschnitt knicken die Spreizstäbe beim Expandieren bzw. Komprimieren der Spreizstruktur definiert, während von den im Wesentlichen geraden zweiten Abschnitten die Spreizkräfte als Druckkräfte bis zu dem erfindungsgemäßen Bereich mit verringerter Biegesteifigkeit übertragen werden. Die im Wesentlichen geraden Abschnitte biegen bzw. knicken bei der erfindungsgemäßen Spreizstruktur also im Wesentlichen nicht und führen daher zu einer besonders großen radialen Aufweitung der Spreizstruktur.

Um eine ausreichend stabile und zugleich als Filterkörbchen geeignete Spreizstruktur zu schaffen, sind besonders vorteilhaft insgesamt sechs Spreizstäbe vorgesehen, die über den Umfang der Spreizstruktur regelmäßig verteilt angeordnet sind.

Nachfolgend werden bevorzugte Ausführungsbeispiele einer erfindungsgemäßen Spreizstruktur anhand der beigefügten schematischen Zeichnungen näher erläutert. Es zeigt:
Fig. 1 eine Draufsicht einer ersten Ausführungsform einer erfindungsgemäßen Spreizstruktur,
Fig. 2 die Seitenansicht II - II gemäß Fig. 1,
Fig. 3 eine perspektivische Ansicht der Spreizstruktur gemäß Fig. 1 an einer Vorrichtung zum Expandieren und Komprimieren der Spreizstruktur,

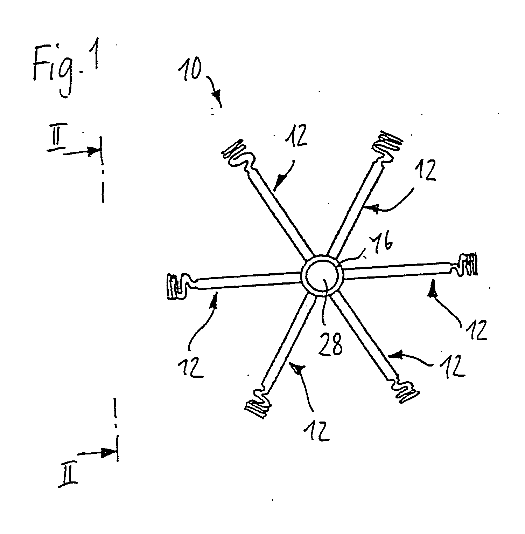

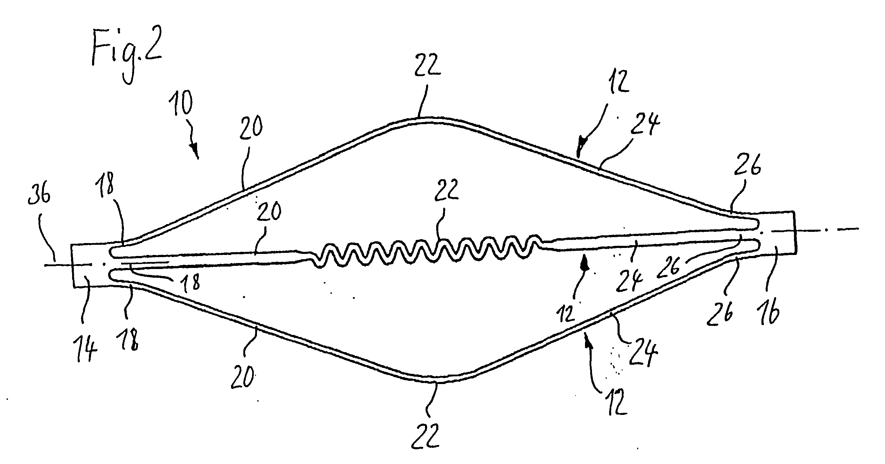

In den Fig. 1 und 2 ist eine Spreizstruktur 10 veranschaulicht, die in ihrer Ausgangsstellung bzw. Ruhelage eine längliche bzw. nicht gespreizte Form aufweist und die in ein Hohlorgan, wie beispielsweise Herz, Blutgefäße, Gallenwege, ableitende Harnwege, Magen-Darm-Trakt, Uterus und Hirnventrikel, einsetzbar ist und dort sowohl als Stützorgan als auch als Fang- oder Filterkörbchen dienen kann. Ferner kann die Spreizstruktur 10 als Metallelektrode dienen, mittels der an einem Hohlorgan Wärme eingebracht werden kann.

Die Spreizstruktur 10 ist im Wesentlichen als länglicher Körper mit insgesamt sechs Spreizstäben 12 gestaltet, die sich in Längsrichtung erstrecken und über den Umfang des Körpers gleichmäßig verteilt angeordnet sind. Vorteilhaft ist eine Gestaltung mit zwischen 2 und 10 Spreizstäben, besonders vorteilhaft sind zwischen 4 und 8 Spreizstäbe vorgesehen.

An den beiden Enden der Spreizstruktur 10 sind zentral ein erster Verbindungsabschnitt 14 bzw. ein zweiter Verbindungsabschnitt 16 ausgebildet. Die Verbindungsabschnitte 14 und 16 sind bevorzugt als zylindrische Hohlkörper bzw. Rohre gestaltet, mit deren Wand die Spreizstäbe 12 jeweils verbunden sind.

Die einzelnen Spreizstäbe 12 sind ausgehend von dem ersten Verbindungsabschnitt 14 jeweils mit einem radial nach außen gebogenen ersten Abschnitt 18 gestaltet, an den ein im Wesentlichen gerader Abschnitt 20 anschließt. Der gerade Abschnitt 20 geht in einen dritten Abschnitt bzw. Bereich 22 über, der mäanderförmig geformt ist, wobei unter Mäanderform die bereits oben definierte Form verstanden wird. An diesen Abschnitt 22 schließt ein im Wesentlichen gerader vierter Abschnitt 24 an, der seinerseits in einen gebogenen fünften Abschnitt 26 übergeht. Der fünfte Abschnitt 26 endet am zweiten Verbindungsabschnitt 16.

Der zweite und der vierte Abschnitt 20 bzw. 24 sind bezogen auf die Längsachse in einem Winkel von 20° zur Längsachse 36 geneigt. Bevorzugt werden Winkel zwischen 10° und 80°, am bevorzugtesten sind bei einer Spreizstruktur 10 mit länglicher bzw. nicht gespreizter Form in seiner Ruhelage Winkel zwischen 15° und 25°. Auf diese Weise ist eine Anordnung geschaffen, bei der die Summe der Längen der einzelnen Abschnitte 18, 20, 22, 24 und 26 größer ist als die Entfernung bzw. der Abstand entlang der Längsachse 36 zwischen den Verbindungsabschnitten 14 und 16.

Die Lage und die eleastischen Eigenschaften der einzelnen Abschnitte 20 bis 26 sind durch die Art und die Stärke des verwendeten Materials beeinflußt. Ferner sind diese Eigenschaften auch durch eine gezielte Wärmebehandlung beeinflußt worden, die auf einzelne dieser Abschnitte angewendet worden ist. Mit anderen Worten ist die dargestellte Spreizstruktur 10 mit dieser Wärmebehandlung in einen Ausgangszustand vorgeformt worden, bei dem die Spreizstäbe 12 bereits vorgebogen sind.

Der dritte Abschnitt 22 ist bei dem dargestellten Ausführungsbeispiel mäanderförmig, derart, dass er im Vergleich zu den benachbarten zweiten und vierten Abschnitten 20 und 24 eine verringerte Biegesteifigkeit aufweist. Die Mäanderform ist dabei mit kurzen Bögen und dazwischenliegenden kurzen Geraden gebildet, die jeweils im Querschnitt im Wesentlichen quadratisch sind. Die restlichen Abschnitte 18, 20, 24 und 26 sind hingegen im Querschnitt rechteckig gestaltet, wobei jeweils die Fläche ihrer Querschnitte größer als die Fläche des Querschnitts im dritten Abschnitt 22 ist. Bei den im Querschnitt rechteckigen zweiten und vierten Abschnitten 20 und 24 ist die längere Seite des Rechtecks radial nach außen gerichtet angeordnet.

Insgesamt sind in dem mäanderförmigen Abschnitt 22 ca. 10 Mäander ausgebildet. Vorteilhaft ist eine Gestaltung mit zwischen 5 und 15 Mäandern, insbesondere vorteilhaft sind zwischen 8 und 12 Mäandern. Der dritte Abschnitt 22 mit der verringerten Biegesteifigkeit ist im Wesentlichen gleich lang bemessen wie die zweiten und vierten Abschnitte 20 und 24. Im Verhältnis zu diesen Abschnitten 20, 22 und 24 sind die gebogenen Abschnitte 18 und 26 kurz gestaltet. Die Amplitude der Mäander des dritten Abschnitts 22 ist etwa doppelt so groß wie die Breite der benachbarten Abschnitte 20 und 24. Vorteilhaft ist eine Amplitude die etwa der 1,5- bis 2,5-fachen Breite der benachbarten Abschnitte 20 und 24 entspricht. Besonders vorteilhaft ist eine Amplitude die etwa der 1,7- bis 2,3-fachen Breite entspricht. Die Dicke der Mäander entspricht dabei vorzugsweise der Dicke der benachbarten Abschnitte 20 und 24.

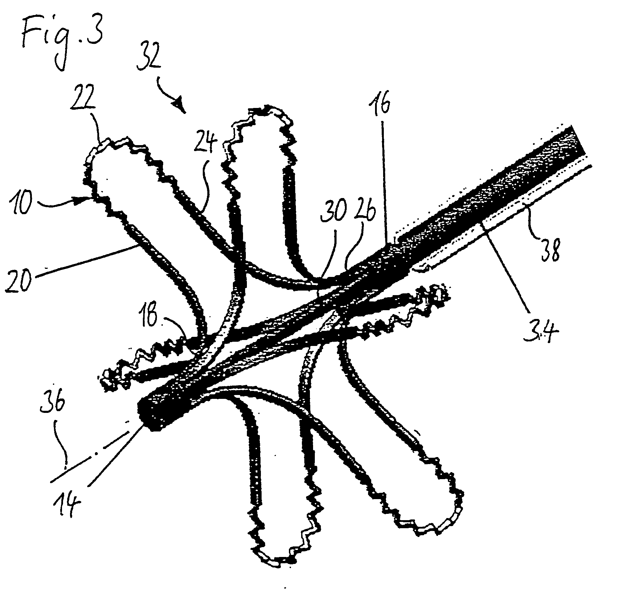

Im ersten und zweiten Verbindungsabschnitt 14 und 16 sind jeweils Durchgangsöffnungen 28 ausgebildet, durch die eine Stange oder ein Stab bzw. ein Rohr oder eine Kanüle 30 einer Expansions- und Kompressionsvorrichtung bzw. Spreizvorrichtung 32 eingeschoben werden kann, wie sie in Fig. 3 veranschaulicht ist. Die Verbindungsabschnitte 14 und 16 sind ferner bevorzugt mit einer Wandstärke versehen, die größer ist als die radiale Dicke der Spreizstäbe 12.

Von der Spreizvorrichtung 32 ist der Stab 30 an dem Verbindungsabschnitt 14 befestigt und ferner von einem Rohr 34 umgeben, das an den Verbindungsabschnitt 16 geschoben werden kann bzw. an diesem befestigt ist.

Mit der Spreizvorrichtung 32 kann der Abstand entlang der Längsachse 36 der Spreizstruktur 10 zwischen den beiden Verbindungsabschnitten 14 und 16 verändert und dadurch die Spreizstruktur 10 expandiert oder komprimiert werden. Dabei wirken zwischen den Verbindungsabschnitten 14 und 16 auf die einzelnen Spreizstäbe 12 bei der dargestellten Spreizvorrichtung 32 Druckkräfte, die diese Spreizstäbe 12 zusammendrängen, wodurch der dritte Abschnitt 22 radial nach außen bewegt und geknickt bzw. gebogen wird. Die Spreizstäbe 12 verformen sich elastisch, sie bewegen sich in Ihrer Mitte wieder radial nach innen, wenn der Druck durch das Rohr 34 verringert oder gelöst wird.

Die Spreizvorrichtung 32 und die Spreizstruktur 10 sind insgesamt in eine Umhüllung 38 eingeschoben, mittels der sie in ein Hohlorgan eingebracht werden können. In dem Hohlorgan wird die Spreizstruktur 10 mittels des Stabes 30 aus der Umhüllung 38 herausgeschoben, so dass die Spreizstruktur 10 mit dem Rohr 34 dann an die Wand des Hohlorgans angespreizt werden kann.

Alternativ kann die Spreizstruktur in ihrer Ruhestellung bereist gespreizt sein und mittels einer Vorrichtung zusammengezogen werden, die zwischen den Verbindungsabschnitten eine Zugkraft in Längsrichtung der Spreizstruktur erzeugt.

Beim Verformen werden aufgrund der verhältnismäßig geringen Biegesteifigkeit der dritten Abschnitte 22 diese Abschnitte besonders leicht und zugleich besonders stark verformt, während die im Wesentlichen geraden Abschnitte 20 und 24 verhältnismäßig stabil sind und nahezu nicht verformt sondern nur insgesamt radial nach außen bzw. nach innen geschwenkt werden. Daher "knicken" die Spreizstäbe 12 beim Expandieren- bzw. Komprimieren an den dritten Abschnitten 22 und können insgesamt in einem Winkel von bis zu 180° gebogen werden. Die beiden Abschnitte 20 und 24 gelangen in eine in Fig. 3 veranschaulichte Stellung, in der sie sich im Wesentlichen parallel erstrecken. In dieser Stellung ist die Spreizstruktur 10 maximal expandiert.

### Bezugszeichenliste

- 10: Spreizstruktur
- 12: Spreizstäbe
- 14: erster Verbindungsabschnitt
- 16: zweiter Verbindungsabschnitt
- 18: erster Abschnitt
- 20: zweiter Abschnitt
- 22: dritter Abschnitt
- 24: vierter Abschnitt
- 26: fünfter Abschnitt
- 28: Durchgangsöffnung
- 30: Stab
- 32: Spreizvorrichtung
- 34: Rohr
- 36: Längsachse
- 38: Umhüllung

## Patentansprüche

1. Spreizstruktur (10) zum Einführen in ein Hohlorgan, mit Spreizstäben (12), die sich ausgehend von einem ersten Verbindungsabschnitt (14) im Wesentlichen in einer Längsrichtung (36) der Spreizstruktur (10) bis zu einem zweiten Verbindungsabschnitt (16) erstrecken, über den Umfang der Spreizstruktur (10) verteilt angeordnet sind und durch radiales Aufspreizen an eine Wand des Hohlorgans anlegbar sind, wobei die Spreizstäbe (10) in Längsrichtung zumindest einen Bereich (22) aufweisen, der im Vergleich zu benachbarten Bereichen(20, 24) eine verringerte Biegesteifigkeit und einen nicht linearen Stababschnitt aufweist, **dadurch gekennzeichnet, dass** der nicht lineare Stababschnitt im kollabierten Zustand bereits Biegungen und Knicke aufweist.

2. Spreizstruktur nach Anspruch 1, **dadurch gekennzeichnet, dass** der mindestens eine Bereich (22) mit verringerter Biegesteifigkeit im Vergleich zu den benachbarten Bereichen (20,24) mit einer verringerten Querschnittsfläche ausgebildet ist.

3. Spreizstruktur nach Anspruch 1 oder 2 , **dadurch gekennzeichnet, dass** der mindestens eine Bereich (22) mit verringerter Biegesteifigkeit mit einem mäanderförmigen Stababschnitt gestaltet ist.

4. Spreizstruktur nach einem der Ansprüche 1 bis 3 , **dadurch gekennzeichnet, dass** der mindestens eine Bereich mit verringerter Biegesteifigkeit mit einem wellenförmigen Stababschnitt gestaltet ist.

5. Spreizstruktur nach einem der Ansprüche 1 bis 4 , **dadurch gekennzeichnet, dass** der mindestens eine Bereich mit verringerter Biegesteifigkeit mit einem zickzackförmigen Stababschnitt gestaltet ist.

6. Spreizstruktur nach einem der Ansprüche 1 bis 5, **dadurch gekennzeichnet, dass** der mindestens eine Bereich (22) mit verringerter Biegesteifigkeit im Querschnitt quadratisch gestaltet ist.

7. Spreizstruktur nach einem der Ansprüche 1 bis 6, **dadurch gekennzeichnet, dass** die Verbindungsabschnitte (14,16) als zentrale Knotenpunkte der Spreizstäbe (12) im Bereich der Längsachse (36) der Spreizstruktur (10) ausgebildet sind.

8. Spreizstruktur nach einem der Ansprüche 1 bis 7 , **dadurch gekennzeichnet, dass** in mindestens einem der Verbindungsabschnitte(16) eine Öffnung (28) ausgebildet ist, durch die hindurch ein zentraler Stab (30) bis zu dem zweiten Verbindungsabschnitt (14) hindurchgeführt werden kann.

9. Spreizstruktur nach einem der Ansprüche 1 bis 8 , **dadurch gekennzeichnet, dass** die Verbindungsabschnitte(14, 16) im Wesentlichen zylindrisch gestaltet sind.

10. Spreizstruktur nach einem der Ansprüche 1 bis 9 , **dadurch gekennzeichnet, dass** im Ausgangszustand der Spreizstruktur (10) die Spreizstäbe (12) jeweils ausgehend von einem Verbindungsabschnitt (14,16) einen radial nach außen gebogenen ersten Abschnitt (18,26) und einen nachfolgenden im Wesentlichen geraden zweiten Abschnitt (20,24) aufweisen.

11. Spreizstruktur nach einem der Ansprüche 1 bis 10 , **dadurch gekennzeichnet, dass** sechs Spreizstäbe (12) vorgesehen sind, die insbesondere über den Umfang der Spreizstruktur (10) regelmässig verteilt angeordnet sind.

12. Spreizvorrichtung mit einer Spreizstruktur (10) nach einem der Ansprüche 1 bis 11 und mit einem zentralen Stab (30), der durch die Spreizstruktur (10), insbesondere durch einen ersten der Verbindungsabschnitte (16) hindurchgeführt und an dem gegenüberliegenden zweiten Verbindungsabschnitt (14) befestigt ist, und mit einem Rohr (34), das den zentralen Stab (30) umgibt und mittels dem der erste Verbindungsabschnitt (16) in Längsrichtung des zentralen Stabes (30) auf diesem verschoben werden kann.

13. Spreizvorrichtung nach Anspruch 12 , **gekennzeichnet durch** eine Umhüllung (38), in die die Spreizstruktur (10), der zentrale Stab (30) und das Rohr (34) einschoben werden können, wobei die Spreizstruktur (10) während oder nach ihrem Herausschieben aus der Umhüllung (38) geöffnet werden kann.

## Claims

1. Expanding structure (10) for introduction into a hollow member, comprising expanding rods (12), which, starting from a first connecting portion (14), extend substantially in a longitudinal direction (36) of the expanding structure (10) up to a second connecting portion (16), are arranged distributed over the periphery of the expanding structure (10) and can be applied, by radial expansion, to a wall of the hollow member, wherein the expanding rods (10) have in the longitudinal direction at least one region (22) which, in comparison to adjacent regions (20, 24), has a reduced flexural rigidity and a non-linear rod portion, **characterized in that** the non-linear rod portion, in the collapsed state, already has bends and kinks.

2. Expanding structure according to Claim 1, **characterized in that** the at least one region (22) of reduced flexural rigidity in comparison with the adjacent regions (20, 24) is configured with a reduced cross-sectional area.

3. Expanding structure according to Claim 1 or 2, **characterized in that** the at least one region (22) of reduced flexural rigidity is shaped with a winding rod portion.

4. Expanding structure according to one of Claims 1 to 3, **characterized in that** the at least one region of reduced flexural rigidity is shaped with a wavy rod portion.

5. Expanding structure according to one of Claims 1 to 4, **characterized in that** the at least one region of reduced flexural rigidity is shaped with a zigzagging rod portion.

6. Expanding structure according to one of Claims 1 to 5, **characterized in that** the at least one region (22) of reduced flexural rigidity is square-shaped in cross section.

7. Expanding structure according to one of Claims 1 to 6, **characterized in that** the connecting portions (14, 16) are configured as central nodal points of the expanding rods (12) in the region of the longitudinal axis (36) of the expanding structure (10).

8. Expanding structure according to one of Claims 1 to 7, **characterized in that** in at least one of the connection portions (16) an opening (28) is configured, through which a central rod (30) can be run through to the second connecting portion (14).

9. Expanding structure according to one of Claims 1 to 8, **characterized in that** the connection portions (14, 16) are shaped substantially cylindrically.

10. Expanding structure according to one of Claims 1 to 9, **characterized in that**, in the initial state of the spreading structure (10), the expanding rods (12), respectively starting from a connecting portion (14, 16), have a first portion (18, 26), which is bent radially outwards, and a following second portion (20, 24), which is substantially straight.

11. Expanding structure according to one of Claims 1 to 10, **characterized in that** six expanding rods (12) are provided, which, in particular, are arranged evenly distributed over the periphery of the expanding structure (10).

12. Expanding apparatus having an expanding structure (10) according to one of Claims 1 to 11, and having a central rod (30), which is run through the expanding structure (10), in particular through a first of the connecting portions (16), and is fastened to the opposite, second connecting portion (14), and having a tube (34), which surrounds the central rod (30) and by means of which the first connecting portion (16), in the longitudinal direction of the central rod (30), can be displaced on the latter.

13. Expanding apparatus according to Claim 12, **characterized by** a sheath (38), into which the expanding structure (10), the central rod (30) and the tube (34) can be inserted, wherein the expanding structure (10) can be opened during or after its expulsion from the sheath (38).

## Revendications

1. Structure d'écartement (10) pour l'introduction dans un organe creux, avec des barrettes d'écartement (12) qui s'étendent à partir d'une première section de liaison (14) essentiellement dans une direction longitudinale (36) de la structure d'écartement (10) jusqu'à une seconde section de liaison (16), et qui sont disposées de façon répartie sur la périphérie de la structure d'écartement (10) et peuvent être appliquées par écartement radial sur une paroi de l'organe creux, les barrettes d'écartement (10) présentant dans la direction longitudinale au moins une zone (22) qui, par rapport aux zones limitrophes (20, 24) présentent une rigidité à la flexion réduite et une section de barrette non linéaire, **caractérisée en ce que** la section de barrette non linéaire présente, à l'état affaissé, déjà des flexions et des plis.

2. Structure d'écartement selon la revendication 1, **caractérisée en ce que** la au moins une zone (22) est configurée avec une rigidité à la flexion réduite par rapport aux zones limitrophes (20, 24) avec une surface de section transversale réduite.

3. Structure d'écartement selon la revendication 1 ou 2, **caractérisée en ce que** la au moins une zone (22) avec une rigidité à la flexion réduite est configurée avec une section de barrette formant des méandres.

4. Structure d'écartement selon l'une des revendications 1 à 3, **caractérisée en ce que** la au moins une zone avec une rigidité à la flexion réduite est configurée avec une section de barrette ondulée.

5. Structure d'écartement selon l'une des revendications 1 à 4, **caractérisée en ce que** la au moins une zone avec une rigidité à la flexion réduite est configurée avec une section de barrette en zigzag.

6. Structure d'écartement selon l'une des revendications 1 à 5, **caractérisée en ce que** la au moins une zone (22) avec une rigidité à la flexion réduite est configurée en section transversale carrée.

7. Structure d'écartement selon l'une des revendications 1 à 6, **caractérisée en ce que** les sections de liaison (14, 16) sont configurées en tant que points nodaux centraux des barrettes d'écartement (12) dans la région de l'axe longitudinal (36) de la structure d'écartement (10).

8. Structure d'écartement selon l'une des revendications 1 à 7, **caractérisée en ce que**, dans au moins l'une des sections de liaison (16), est ménagée une ouverture (28) à travers laquelle peut être introduite une barrette centrale (30) jusqu'à la seconde section de liaison (14) .

9. Structure d'écartement selon l'une des revendications 1 à 8, **caractérisée en ce que** les sections de liaison (14, 16) sont configurées essentiellement de façon cylindrique.

10. Structure d'écartement selon l'une des revendications 1 à 9, **caractérisée en ce que**, à l'état de départ de la structure d'écartement (10), les barrettes d'écartement (12), en partant respectivement d'une section de liaison (14, 16), présentent une première section (18, 26) pliée vers l'extérieur et une seconde section suivante essentiellement droite (20, 24).

11. Structure d'écartement selon l'une des revendications 1 à 10, **caractérisée en ce qu'**il est prévu six barrettes d'écartement (12) qui sont agencées en particulier sur la périphérie de la structure d'écartement (10) et sont réparties de façon régulière.

12. Dispositif d'écartement avec une structure d'écartement (10) selon l'une des revendications 1 à 11 et avec une barrette centrale (30) qui est traversée par la structure d'écartement (10), en particulier par une première des sections de liaison (16), et qui est fixée sur une seconde section de liaison (14) opposée, et avec un tube (34) qui entoure la barrette centrale (30) et peut être poussé sur celle-ci au moyen de la première section de liaison (16) dans la direction longitudinale de la barrette centrale (30).

13. Dispositif d'écartement selon la revendication 12, **caractérisé par** une enveloppe (38) dans laquelle la structure d'écartement (10), la barrette centrale (30) et le tube (34) peuvent être introduits, la structure d'écartement (10) pouvant être ouverte pendant ou après son extraction de l'enveloppe (38).
